(19) Europäisches Patentamt
European Patent Office
Office européen des brevets

(11) Publication number: **0 042 750**
**B1**

(12) # EUROPEAN PATENT SPECIFICATION

(45) Date of publication of patent specification: **30.04.86**

(21) Application number: **81302801.6**

(22) Date of filing: **22.06.81**

(51) Int. Cl.$^4$: **C 07 D 241/44**,
C 07 D 241/52,
C 07 D 413/12, A 01 N 43/60,
A 01 N 43/76 // C07C69/736

(54) **Quinoxalinyloxy ethers as selective weed control agents.**

(30) Priority: **23.06.80 US 161940**
**03.04.81 US 250132**

(43) Date of publication of application:
**30.12.81 Bulletin 81/52**

(45) Publication of the grant of the patent:
**30.04.86 Bulletin 86/18**

(84) Designated Contracting States:
**AT CH GB IT LI LU SE**

(56) References cited:
**EP-A-0 000 483**
**EP-A-0 004 414**
**EP-A-0 023 785**
**EP-A-0 024 907**
**EP-A-0 026 622**
**EP-A-0 046 767**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Fawzi, Maged Mohamed**
**26 Glenbarry Drive**
**Wilmington Delaware 19808 (US)**

(74) Representative: **Hildyard, Edward Martin et al**
**Frank B. Dehn & Co. Imperial House**
**15-19 Kingsway**
**London WC2B 6UZ (GB)**

(56) References cited:
**EP-A-0 046 768**
**GB-A-1 548 847**
**GB-A-2 042 539**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Technical Field

This invention relates to quinoxalinyloxy ethers which are useful as selective weed control agents. The compounds are especially useful for controlling grass weeds in broadleaf crops such as soybeans.

The presence of undesirable vegetation causes substantial damage to useful crops, especially agricultural products that satisfy man's basic food and fiber needs, such as cotton, soybeans, beans and the like. The current population explosion and concomitant world food and fiber shortage underlie the need for improvements in the efficiency of producing these crops. Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

Herbicidal compounds related in structure to those of the present invention are disclosed in EP—A—23,785; GB—A—2042,539; EP—A—46,767; and EP—A—46,768. The compounds of this invention show an unexpected herbicidal selectivity compared to the prior art compounds closest in structure.

Summary of the Invention

This invention relates to novel compounds of formula I:

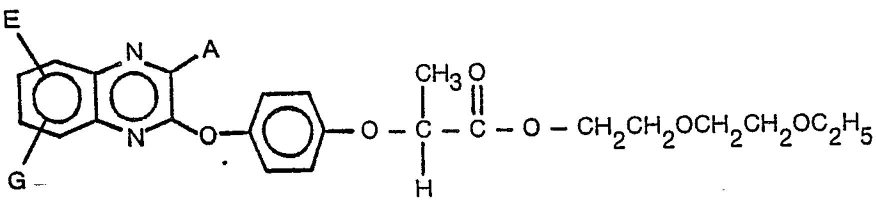

wherein

A is H or $C_1$—$C_4$ alkyl;

E is H, Cl, Br, F, $CF_3$, $CH_3$ or $OCH_3$;

G is H or Cl;

provided that:

(a) when E is other than H or Cl, then G is H;

(b) when E or G are other than H, then A is H or $CH_3$.

Preferred in order of increasing activity and/or increasingly favorable ease of synthesis, are those compounds of formula I wherein:

(1) A is H;

(2) In addition to (1) G is H or Cl and E is H, Cl, F or $CF_3$, provided that when G is chlorine, E must be chlorine and they are in the 6- and 7-positions;

(3) In addition to (1) G is H and E is H, Cl, F or $CF_3$;

(4) In addition to (3) E is in the 6-position.

Specifically preferred by reason of high activity and/or ease of synthesis is the following compound: 2-(2-ethoxyethoxy)ethyl 2-[4-(6-chloroquinoxalin-2-yloxy)phenoxy]propanoate.

Detailed Description of the Invention

This invention relates to compounds of formula I, to herbicidal compositions containing compounds of formula I and to methods of using these novel compounds to control undesirable plant growth.

Synthesis

The compounds of formula I can be prepared in a number of ways, for example by:

(a) reacting an acid chloride of formula

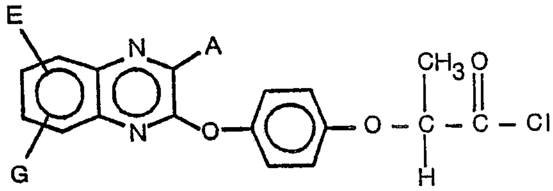

(IIc)

wherein A, E and G are as defined above, with 2-(ethoxyethoxy)ethanol in the presence of an equimolar amount of an acid acceptor; or

(b) reacting an acid of formula

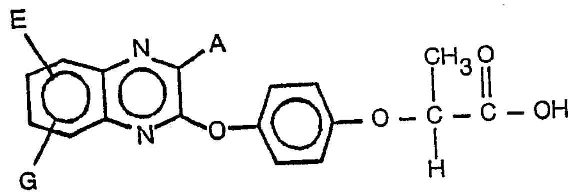

(IIb)

wherein A, E and G are as defined above, with a 1-halo-2-(ethoxyethoxy)ethane in the presence of a phase transfer catalyst.

In process (a), suitable acid acceptors include, but are not limited to, pyridine and N,N-dimethylaniline. The reaction is preferably run under a nitrogen atmosphere at a temperature of from about 0° to about 40°C.

The acid chloride of formula IIc may be prepared by standard procedures, e.g. by treating the acid of formula IIb with excess thionyl chloride; or by treating the sodium salt of the acid of formula IIb with oxalyl chloride.

Acids of formula IIb may be made by hydrolysis of an ester of formula IIa

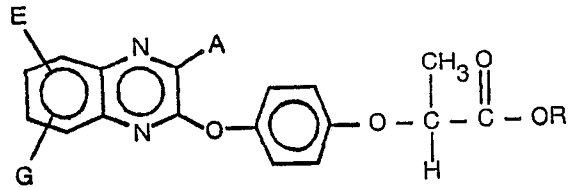

(IIa)

where R is $C_1$—$C_4$ alkyl.

Either acid hydrolysis with, for example, dilute hydrochloric or sulfuric acid, or basic hydrolysis with, for example, sodium hydroxide or potassium hydroxide, followed by acidification with dilute acids yields the acids of formula IIb.

The esters of formula IIc may be made by combining, preferably in equimolar amounts, a 2-haloquinoxaline and the alkali metal salt of the alkyl 2-(4-hydroxyphenyl)-alkanoate as illustrated below:

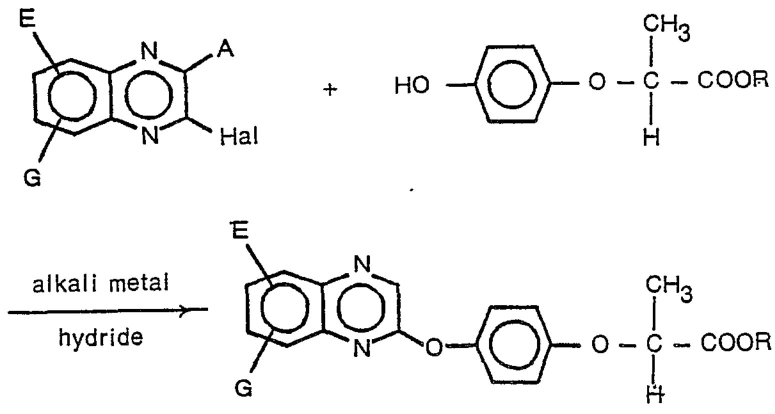


(IIa)

where A, E, G and R are as previously defined.

Suitable solvents for this reaction include dimethylformamide, dimethylsulfoxide, diglyme and methylethylketone. The reaction is preferably carried out at a temperature between about 25 and about 130°C. The esters of Formula IIa are useful starting materials for preparing compounds of this invention.

The 2-chloroquinoxalines used in this reaction can be prepared by methods known in the art. Glyoxylic acid, substituted glyoxylic acid or an alkyl ester of glyoxylic acid is combined with a substituted *o*-phenylenediamine in ethanol to yield a 2-hydroxyquinoxaline, as described in JACS *71* 6 (1949). The 2-hydroxyquinoxaline is treated with phosphorous oxychloride as described in JCS, 519 (1948) or *Bull. Soc. Chem. France,* 356 (1963), to give the desired 2-chloroquinoxalines. The disclosures of the above-cited references are hereby incorporated by reference.

The alkyl 2-(4-hydroxyphenoxy)alkanoates used in the reaction are known in the art and can be prepared in a two-step process from commercially available compounds. First, 4-benzyloxyphenol is alkylated by reaction with an alkyl bromoalkanoate. The product is hydrogenated in the presence of a palladium over carbon catalyst to yield the desired compound.

The following examples illustrate the preparation of intermediates for synthesizing compounds of this invention.

Example 1

Methyl 2-[4-(2-quinoxalinyloxy)phenoxy]propanoate

In a nitrogen atmosphere, a solutioin of 5.9 g (0.03 mole) methyl 2-(4-hydroxyphenoxy)propanoate in 20 cc of dimethylformamide was added dropwise at about 15°C to 1.5 g (0.03 mole) 57% sodium hydride in 20 cc of dimethylformamide. When the evolution of hydrogen ceased, 4.9 g (0.03 mole) 2-chloroquinoxaline was added and the reaction mixture was heated at 130°C for 4 hours. After standing overnight at room temperature, the reaction mixture was poured into about 100 cc of cold water. The precipitated solid was filtered and crystallized from methanol to give 4.2 g of product, m.p. 110—115°.

NMR (DMSO)δ:

1.5 (d, J = 7Hz, 3H);
3.7 (s, 3H);
4.9 (q, J = 7Hz, 1H);
7.15 (ABq, J = 9Hz, 4H);
7.5—8.2 (m, 4H);
8.9 (s, 1H).

Example 2

Methyl 2-[4-(6,7-dichloro-2-quinoxalinyloxy)phenoxy]-propanoate

In a nitrogen atmosphere, a solution of 3.9 g (0.02 mole) methyl 2-(4-hydroxyphenoxy)propanoate in 20 cc of dimethylformamide was added dropwise at about 15°C to a suspension of 0.8 g sodium hydride in 10 cc dimethylformamide. When the evolution of hydrogen ceased, 4.7 g (0.02 mole) 2,6,7-trichloroquinoxaline was added and the reation mixture was heated for 5 hours at 130°C. Filtering the reaction mixture yielded a small amount of insoluble material. The filtrate was poured into approximately 200 cc of ice-water. Sodium chloride was added to the solution which was then extracted with 1200 cc of ether (4 × 300). The ethereal extracts were combined and dried over magnesium sulfate. The ether was removed under vacuum an the crude product was crystallized from $CH_3OH$. Yield 2.8 g, m.p. 100—105°.

NMR ($CDCl_3$)δ:

1.7 (d, J = 7Hz, 3H);
3.8 (s, 3H);
4.75 (q, J = 7Hz, 1H);
7.15 (ABq, J = 9Hz, 4H);
8 (s, 1H);
8.3 (s, 1H);
8.8 (s, 1H).

The following compounds were also prepared using the basic procedure described in this example.

Methyy 2-[4-(6 or 7-chloro-2-quinoxalinyloxy)phenoxy]-propanoate, (mixture of 6- and 7-isomers), m.p. 108—111°C.

NMR ($CDCl_3$)δ:

1.6 (d, J = 7Hz, 3H);
3.7 (s, 3H);
4.7 (q, J = 7Hz, 1H);
7.1 (ABq, J = 9Hz, 4H);
7.6—7.7 (m, 2H);
8—8.1 (m, 1H);
8.7 (s, 1H).

Methyl 2-[4-(6-trifluoromethyl-2-quinoxalinyloxy)-phenoxy]propanoate and its 7-trifluoromethyl isomer.

NMR ($CDCl_3$)δ:

1.7 (d, J = 7Hz, 3H);
3.8 (s, 3H);
4.75 (q, J = 7Hz, 1H);
7.15 (ABq, 9Hz, 4H);
7.4—8.5 (m, 3H);
8.85 and 8.95 (2 singlets, 1H).

Methyl 2-[4-(6 or 7-fluoro-2-quinoxalinyloxy)phenoxy]-propanoate, m.p. 113—116°C.

NMR ($CDCl_3$)δ:

1.65 (d, J = 7Hz 3H);
3.75 (s, 3H);
4.7 (q, J = 7Hz, 1H);
7.15 (ABq, J = 9Hz, 4H);
7.35—8 (m, 3H);
8.85 (q, 1H).

Following the procedures of Examples 1 and 2, and by substituting the appropriate 2-chloroquinoxaline and alkyl 2-(4-hydroxyphenoxy)alkanoate, the compounds listed in Table I can be prepared.

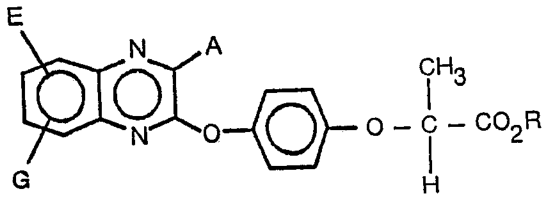

TABLE 1

| A | E | G | R |
|---|---|---|---|
| $CH_3$ | 6-F | H | $CH_3$ |
| H | 6-Br | H | $CH_3$ |
| H | 6-$OCH_3$ | H | $CH_3$ |
| $CH_3$ | H | H | $CH_3$ |
| H | 6-Cl | 7-Cl | $C_2H_5$ |
| H | 7-Cl | H | *n*-$C_4H_9$ |
| $CH_3$ | 6-Cl | H | $CH_3$ |
| $C_2H_5$ | H | H | $CH_3$ |
| $C_3H_7$ | H | H | $CH_3$ |
| $C_4H_9$ | H | H | $C_2H_5$ |
| H | 6-Cl | 8-Cl | $CH_3$ |
| H | 5-Cl | 7-Cl | $CH_3$ |

Example 3

2-[4-(2-Quinoxalinyloxy)phenoxy]propanoic acid

3.2 g (0.01 mole) Methyl 2-[4-(2-quinoxalinyloxy)phenoxy]propanoate was added to a solution of 0.7 g (0.01 mole) potassium hydroxide in 5 cc water and 60 cc methanol. the mixture was stirred at room temperature overnight and then filtered to remove a small amount of insoluble material. The methanol was removed under vacuum. Ice and hydrochloric acid were added to the mixture until it was acidic (pH 2). The precipitated acid was filtered and purified by redissolution into saturated sodium bicarbonate solution, reprecipitation by acidification with dilute hydrochloric acid and crystallization from methanol. Yield 0.7 g, m.p. 137—141°C.

NMR ($CDCl_3$—DMSO)δ:

1.7 (d, J = 7Hz, 3H);
4.7 (q, J = 7Hz, 1H);
7.15 (ABq, J = 9Hz, 4H);
7.45—8.4 (m, 4H); and
8.8 (s, 1H).

Using procedures similar to that above, the compounds listed in Table II can be prepared.

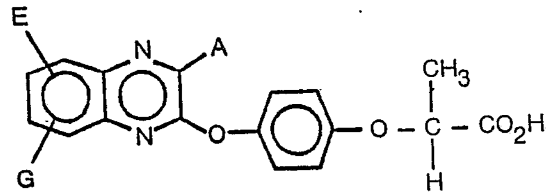

TABLE II

| A | E | G |
|---|---|---|
| $CH_3$ | 6-F | H |
| H | 6-Br | H |
| H | 6-$OCH_3$ | H |
| $CH_3$ | H | H |
| H | 6-CL | 7-CL |
| H | 7-Cl | H |
| $CH_3$ | 6-Cl | H |
| $C_2H_5$ | H | H |
| $C_3H_7$ | H | H |
| $C_4H_9$ | H | H |
| H | 6-Cl | 8-Cl |
| H | 5-Cl | 7-Cl |

Example 4

Synthesis of methyl 2-(4-hydroxyphenoxy)propanoate

A 42 g (0.25 mole) portion of methyl 2-bromopropionate was added to 0.25 mole of the sodium salt of *p*-benzyloxyphenol (prepared from 13.5 g sodium methoxide and 50 g *p*-benzyloxyphenoxy) in 70 cc of DMF. The reaction mixture was heated at approximately 80°C for 14.5 hours. The cooled reaction mixture was then poured into 1 liter ice-water. The product was isolated by ether extraction and was crystallized from methanol to give 45.2 g methyl 2-(4-benzyloxyphenoxy)-propanoate, m.p. 38—42°.

NMR ($CDCl_3$)δ:
1.6 (d, J = 7Hz, 3H);
3.7 (s, 3H);
4.6 (q, J = 7Hz, 1H);
4.9 (s, 2H);
6.8—7 (m, 4H);
7.2—7.6 (m, 5H).

Catalytic hydrogenation at about $3 \times 10^5$ Pa (45 p.s.i.) of 40 g of methyl 2-(4-benzyloxyphenoxy)-propanoate in 200 cc DMF in the presence of 1 g of 10% Pd/C gives methyl 2-(4-hydroxyphenoxy)propanoate in almost quantitative yield. The material is of sufficient purity to be used in the preparation of the compounds of this invention.

NMR ($CDCl_3$)δ:
1.6 (d, J = 7Hz, 3H);
3.7 (s, 3H);
4.6 (q, J = 7Hz, 1H);
6.7—6.9 (m, 4H);
8 (s, 1H).

The intermediates in the foregoing Examples and in Tables I and II may be converted to compounds of formula I by the methods described hereinbefore, and as illustrated by analogy in the following Examples 5 and 6, which illustrate the preparation of esters by processes (b) and (a) respectively).

Thus may be prepared e.g. 2-(2-ethoxyethoxy)ethyl 2-[4-(6- or 7-chloroquinoxaline-2-yloxy)phenoxy] propanoate, as an oil, and likewise the corresponding 3-methyl-6-fluoro compound.

Example 5

Allyl 2-[4-(6- or 7-chloro-2-quinoxalinyloxy)phenoxy]-propanoate

5 cc Allyl bromide was added to a vigorously stirred mixture of 2 g (0.0058 mole) 2-[4-(6 or 7-chloro-2-quinoxalinyloxy)phenoxy]propanoic acid, 0.2 g of tetrabutylammonium hydrogen sulfate and 1 g (0.025 mole of sodium hydroxide in 30 cc of water and 85 cc of chloroform.

The mixture was stirred for 18 hours. The chloroform layer was separated and the volatile matter was removed by evaporation under reduced pressure. The residue was dissolved in ether and the ethereal layer was dried over magnesium sulfate. Removal of the magnesium sulfate by filtration and the ether by evaporation under vacuum gave the crude product. The crude product was purified by dissolving it in a mixture of ether-hexane (3:1) and filtering it through a plug of silica and washing the silica with *n*-hexane.

Concentration of the filtrate and crystallization of the residue from ether-*n*-hexane (1:4) gave 1.3 g of the desired product, m.p. 64—69°C.

NMR ($CDCl_3$)δ:
1.6 (d, J = 7Hz, 3H);
4.5—6.1 (m, 6H);
6.9—8.1 (m, 7H).

Example 6

Allyl 2-[4-(2-quinoxalinyloxy)phenoxy]propanoate

The following procedure can be employed to make the title compound.

To a solution of 1.2 g (0.021 mole) allyl alcohol and 1.6 g (0.02 mole) pyridine in 50 cc methylene chloride, add 6.6 g (0.02 mole) of 2-[4-(2-quinoxalinyloxy)phenoxy]propionyl chloride in 60 cc methylene chloride. Stir the mixture at room temperature overnight. Wash the methylene chloride solution with water and dry the solution over magnesium sulfate concentrate under vacuum to give allyl 2-[4-(2-quinoxalinyloxy)phenoxy]propanoate.

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE III

| | | Weight Percent* | |
|---|---|---|---|
| | Active Ingredient | Diluent(s) | Surfactant(s) |
| Wettable Powders | 20—90 | 0—74 | 1—10 |
| Soil Suspensions, Emulsions, Solutions (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

*Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed.,

Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed grandular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959 Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—113.

For guidance on formulation, reference should also be made to EP—$A_2$—0042750.

Use

The test results to follow suggest that the compounds of the present invention are useful when applied as pre- and/or post-emergence treatments for broad-spectrum control of a wide variety of weed species growing on industrial sites, storage lots, along fences and building foundations, along railroad and utility rights-of-way, etc. In addition, it appears that the compounds of this invention should have utility for weed control in certain crops, such as soybeans, cotton, sugarbeets and beans.

The tested compounds have been shown to be particularly useful for selectively removing and controlling grass weeds, including volunteer corn, in broadleaf crops including soybeans, cotton, sugarbeets, beans, flax, cabbage, tomatoes, potatoes, peanuts, carrots, cucurbits, endive, beets, etc. Grassweeds include crabgrass (*Digitaria* sp.), barnyardgrass (*Echinochloa crusgalli*), wild oats (*Avena fatua*), etc. Some of the compounds also may control certain broadleaved weeds, and some are tolerant to certain grass crops such as rice and wheat. Tests indicate that the compounds of this invention generally show a remarkable and unexpected degree of safety to broadleaf crops and an unusual phytotoxicity to grass weeds whether applied to the soil before weeds and crops emerge, that is, pre-emergence or whether applied post-emergence including spraying on the weeds and crops. In addition, the compounds are useful when applied as pre-emergence or post-emergence treatments alone or in combination with other herbicides or surfactants for broad-spectrum control of a wide variety of weed species growing on industrial sites, on storage lot and along fences, building foundations, railroad, highway and utility rights-of-way, etc.

The precise amount of the compounds of this invention to be used in any particular situation will vary according to the end result desired. Factors affecting the optimum rate of application include the particular compound to be used, the plant species to be controlled, soil type, formulation used, prevailing weather conditions, foliage density, length of time for which residual activity is desired, etc. Broadly speaking, the compounds are used at levels of about 0.005 to 20 kilograms per hectare, with the preferred range being about 0.02 to 10 kilograms per hectare. Not all of the compounds of this invention will be active at the lower levels of the stated application rate ranges. In general, the higher rates of application from within this range will be selected for adverse conditions or where extended persistence in soil is desired, and the lower rates for weed control in crops.

The herbicides effectively control grass weeds as demonstrated by the examples, but they generally do not control broadleaf weeds at low application rates. To obtain control of a wider spectrum of both broadleaf and grass weeds, combination treatments consisting of compounds of this invention with other herbicides effective on broadleaf weeds may be used to advantage. Combination treatments may be used with the components applied simultaneously as in a tank mix or mixed formulation, or sequentially with either or both components applied preplant incorporated, pre-emergence, post-emergence, post-emergence-directed, broadcast, band or spot treatment or any combination of these methods. The following examples of combination utility are cited:

Compounds of this invention with —

| Other Herbicides | Use |
|---|---|
| bentazon (post-) | Soybeans |
| 2,4-DB (post-) | Peanuts, Soybeans, Alfalfa, Clover |
| Simazin (pre-) | Nursery, Citrus, Peaches, Established Alfalfa |
| pyrazon (pre-, early post-) | Sugarbeets |
| silvex | Fence lines, rights of ways |
| dichloroprop (post-) | Brush, Release of Evergreens |
| MCPB (early post-) | peas |
| dicamba (pre-) | flax and rape |
| desmedipham (post-) | Sugarbeets |
| prometryn (pre-) | Celery and Cotton |
| Phenmedipham (post-) | Sugarbeets |
| acifluorfen (Blazer®) (Post-) | Soybeans |
| 2-chloro-N-[(4-methoxy-6-methyl-1,3,5-triazin-2-yl)aminocarbonyl]benzenesulfonamide | flax |
| 1-methylethyl 2[[(4,6-dimethoxy-1,3,5-triazin-2-yl)-aminocarbonyl]]aminosulfonly]]benzoate | flax |
| 1-methylethyl 2[[(4-methoxy-6-methyl-1,3,5-triazin-2-yl)-aminocarbonyl]aminofulfonyl]]-benzoate | flax |
| dinoseb (post-) | Soybeans |
| lenacil (pre-) | Sugarbeets |
| bromoxynil (post-) | Wheat and barley |
| fluometuron (pre-) | Cotton |
| 1-[2(([(4,6-dimethoxypyrimidin-2-yl)aminocarbonyl]aminosulfonyl)) benzoyl]pyrrolidine | Soybeans |
| 2-propenyl(2-[[([4,6-dimethoxypyrimidin-2-yl)aminocarbonyl]-aminosulfonyl]]benzoate | Soybeans |
| pyrazolate | Rice |
| naproanilide | Rice |

## Test A

Seeds of crabgrass (*Digitaria* spp.), barnyard-grass (*Echinochloa crusgalli*), wild oats (*Avena fatua*), cassia (*Cassia tora*), morningglory (*Ipomoea* spp.), cocklebur (*Xanthium* spp.), sorghum, corn, soybean, rice, wheat and nutsedge tubers (*Cyperus rotundus*) were planted in a growth medium and treated pre-emergence with 2-(2-ethoxyethoxy)ethyl 2-[4-(6- or 7-chloro-2-quinoxalinyloxy)phenoxy]propanoate (an oil) dissolved in a non-phytotoxic solvent. At the same time, cotton having five leaves (including cotyledonary ones), bush beans with the third trifoliate leaf expanding, crabgrass with two leaves, barnyardgrass with two leaves, wild oats with one leaf, cassia with three leaves (including cotyledonary ones), morningglory and cocklebur with four leaves (including the cotyledonary ones), sorghum and corn with three leaves, soybean with two cotyledonary leaves, rice with two leaves, wheat with one leaf, and nutsedge with three-five leaves were sprayed. Treated plants and controls were maintained in a

greenhouse for sixteen days. All species were comapred to controls and visually rated for response to treatment seven and sixteen days after treatment. The ratings are based on a numerical scale extending from 0 = no injury, to 10 = complete kill. The accompanying descriptive symbols have the following meanings:

S = albinism;
G = growth retardation
C = chlorosis/necrosis;
E = emergence inhibition
H = formative effects;
U = unusual pigmentation;
B = burn;
X = axillary stimulation; and
6Y = abscised buds or flowers.

The ratings for the compound tested by this procedure are shown in Table A for 16 days after treating. It can be seen that the compound is useful as a selective herbicide in crops such as soybeans, cotton and beans. The rate of application was too high to show selectivity to rice in this test.

TABLE A

| kg/ha | 0.1 |
|---|---|
| Post-Emergence | |
| Bushbean | 1C |
| Cotton | 1B |
| Morningglory | 0 |
| Cocklebur | 0 |
| Cassia | 0 |
| Nutsedge | 0 |
| Crabgrass | 10C |
| Barnyardgrass | 10C |
| Wild Oats | 9C |
| Wheat | 9C |
| Corn | 10C |
| Soybean | 0 |
| Rice | 9C |
| Sorghum | 10C |
| Pre-Emergence | |
| Morningglory | 0 |
| Cocklebur | 0 |
| Cassia | 0 |
| Nutsedge | 3G |
| Crabgrass | 9C |
| Barnyardgrass | 10E |
| Wild Oats | 1C, 9G |
| Wheat | 5C, 9G |
| Corn | 5U, 9G |
| Soybean | 0 |
| Rice | 10E |
| Sorghum | 3C, 9H |

**Claims for the Contracting States: CH GB IT LI LU SE**

1. A compound of the formula

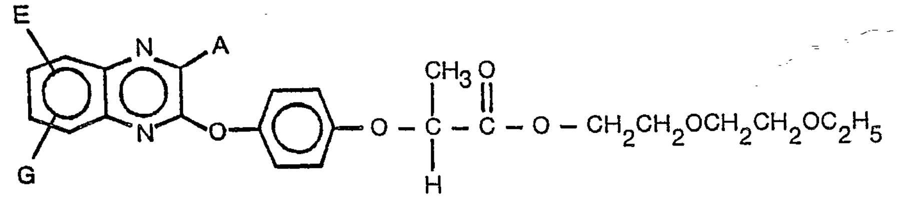

wherein

A is H or $C_1$—$C_4$ alkyl;

E is H, Cl, Br, F, $CF_3$, $CH_3$ or $OCH_3$;
G is H or Cl;
provided that:
(a) when E is other than H or Cl, then G is H;
(b) when E or G are other than H, then A is H or $CH_3$.

2. A compound of claim 1 wherein A is H.

3. A compound of claim 2 wherein G is H and E is H, Cl, F or $CF_3$, or G and E are 6- and 7-Cl.

4. A compound of claim 3 where G is H and E is H, Cl, F or $CF_3$.

5. A compound of claim 4 where E is in the 6-position.

6. A compound of claim 1 which is 2-(2-ethoxyethoxy)ethyl 2-[4-(6-chloroquinoxalin-2-yloxy)pehnoxyl] propanoate.

7. A composition for the control of undesirable vegetation comprising a herbicidally effective amount of a compound of any of claims 1 to 6 and at least one of (a) a surface active agent and (b) a solid or liquid diluent.

8. A method for the control of undesirable vegetation which comprises applying to the locus of said vegetation a herbicidally effective amount of a compound of any of claims 1 to 6.

9. The method of claim 8 wherein said undesirable vegetation is selectively controlled in rice.

10. A process for preparing a compound of any of claims 1 to 6 which comprises
(a) reacting an acid chloride of formula

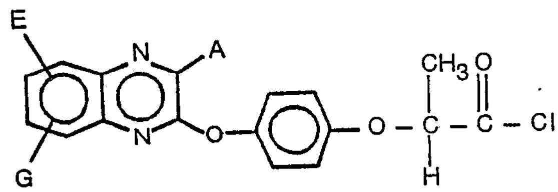

(IIc)

wherein A, E and G are as defined in claim 1, with 2-(ethoxyethoxy)ethanol in the presence of an equimolar amount of an acid acceptor; or
(b) reacting an acid of formula

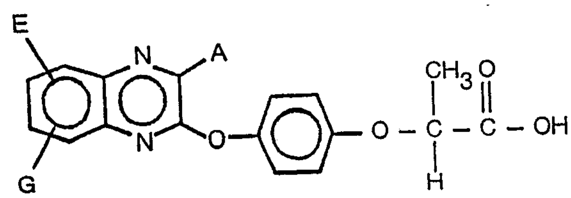

(IIb)

wherein A, E and G are as defined in claim 1, with a 1-halo-2-(ethoxyethoxy)ethane in the presence of a phase transfer catalyst.

**Claims for the Contracting State: AT**

1. A process for the preparation of a compound of the formula

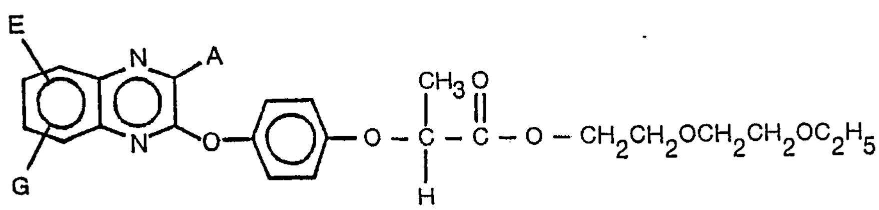

(I)

wherein
A is H or $C_1$—$C_4$ alkyl;
E is H, Cl, Br, F, $CF_3$, $CH_3$ or $OCH_3$;
G is H or Cl;
provided that:
(a) when E is other than H or Cl, then G is H;
(b) when E or G are other than H, then A is H or $CH_3$;
which comprises
(a) reacting an acid chloride of formula

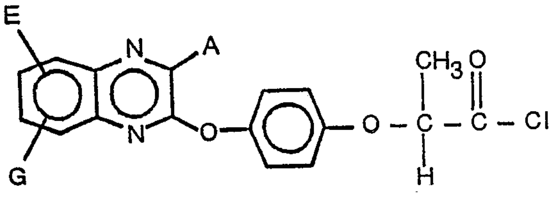

(IIc)

wherein A, E and G are as defined above, with 2-(ethoxyethoxy)ethanol in the presence of an equimolar amount of an acid acceptor; or

(b) reacting an acid of formula

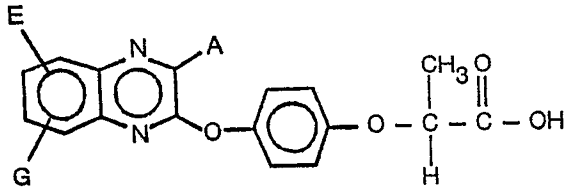

(IIb)

wherein A, E and G are as defined above, with a 1-halo-2-(ethoxyethoxy)ethane in the presence of a phase transfer catalyst.

2. A process of claim 1 wherein A is H.

3. A process of claim 2 wherein G is H and E is H, Cl, F or $CF_3$, or G and E are 6- and 7-Cl.

4. A process of claim 3 where G is H and E is H, Cl, F or $CF_3$.

5. A process of claim 4 where E is in the 6-position.

6. A process of claim 1 wherein the product is 2-(2-ethoxyethoxy)ethyl 2-[4-(6-chloroquinoxalin-2-yloxy) phenoxy]propanoate.

7. A composition for the control of undesirable vegetation comprising a herbicidally effective amount of a compound of formula (I) as defined in any of claims 1 to 6 and at least one of (a) a surface active agent and (b) a solid or liquid diluent.

8. A method for the control of undesirable vegetation which comprises applying to the locus of said vegetation a herbicidally effective amount of a compound of formula (I) as defined in any of claims 1 to 6.

9. The method of claim 8 wherein said undesirable vegetation is selectively controlled in rice.

10. The method of claim 8 or 9 wherein the compound of claim 6 is employed.

**Patentansprüche fur die Vertragsstaaten: CH GB IT LI LU SE**

1. Eine Verbindung mit der Formel

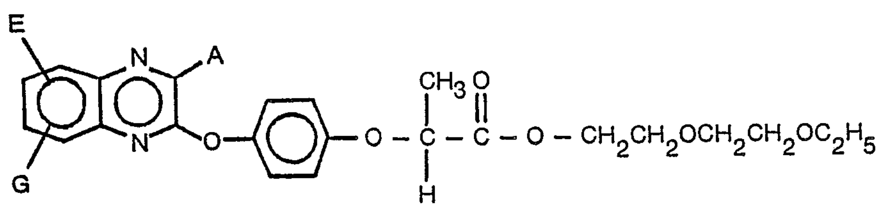

worin

A H oder $C_1$—$C_4$ Alkyl ist;

E H, Cl, Br, F, $CF_3$, $CH_3$ oder $OCH_3$ ist;

G H oder Cl ist;

mit der Maßgabe daß:

(a) wenn E von H oder Cl verschieden ist, dann G H ist;

(b) wenn E oder G von H verschieden sind, dann A H oder $CH_3$ ist.

2. Eine Verbindung nach Anspruch 1, worin A H ist.

3. Eine Verbindung nach Anspruch 2, worin G H ist und E H, Cl, F oder $CF_3$ ist, oder G und E 6- und 7-Cl sind.

4. Eine Verbindung nach Anspruch 3, worin G H ist und E H, Cl, F oder $CF_3$ ist.

5. Eine Verbindung nach Anspruch 4, worin E in der 6-Stellung ist.

6. Eine Verbindung nach Anspruch 1, die 2-(Ethoxyethoxy)ethyl-2-[4-(6-chlorchuinoxalin-2-yloxy)phenoxy]-propanoat ist.

7. Eine Zusammensetzung zur Bekämpfung unerwünschter Vegetation, die eine herbizid wirksame

Menge einer Verbindung nach einem der Ansprüche 1 bis 6 und mindestens eines von (a), ein oberflächenaktives Mittel und (b) ein festes oder flüssiges Verdünnungsmittel, umfaßt.

8. Ein Verfahren zur Bekämpfung unerwünschter Vegetation, das den Auftrag auf den Ort dieser Vegetation von einer herbizid wirksamen Menge einer Verbindung nach einem der Ansprüche 1 bis 6 umfaßt.

9. Das Verfahren nach Anspruch 8, bei dem die unerwünschte Vegetation in Reis selektiv bekämpft wird.

10. Ein Verfahren zur Herstellung einer Verbindung nach einem der Ansprüche 1 bis 6, das umfaßt:

(a) die Reaktion eines Säurechlorids der Formel

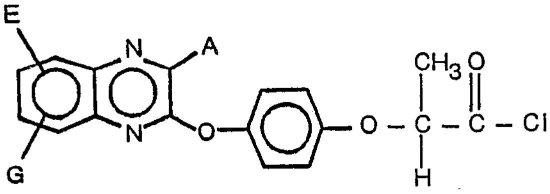

(IIc)

worin A, E und G wie in Anspruch 1 definiert sind, mit 2-(Ethoxyethoxy)ethanol in der Anwesenheit einer äquimolaren Menge eines Säureakzeptors; oder

(b) die Reaktion einer Säure der Formel

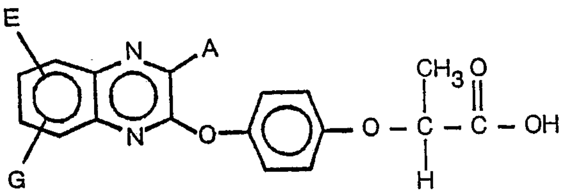

(IIb)

worin A, E und G wie in Anspruch 1 definiert sind, mit einem 1-Halogen-2-(ethoxyethoxy)ethan in Anwesenheit eines Phasentransfer-Katalysators.

**Patentansprüche fur den Vertragsstaat: AT**

1. Ein Verfahren zur Herstellung einer Verbindung der Formel

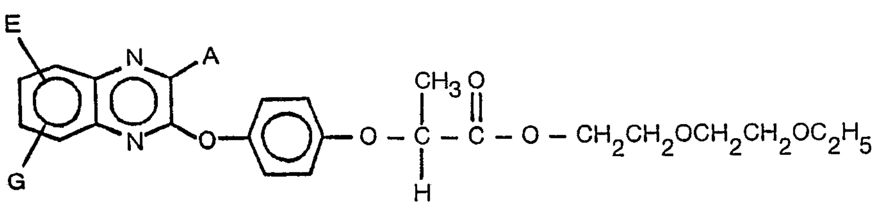

(I)

worin

A H oder $C_1$—$C_4$ Alkyl ist;

E H, Cl, Br, F, $CF_3$, $CH_3$ oder $OCH_3$ ist;

G H oder Cl ist;

mit der Maßgabe daß:

(a) wenn E von H oder Cl unterschiedlich ist, dann G H ist;

(b) wenn E oder G von H unterschiedlich sind, dann A H oder $CH_3$ ist;

welches umfaßt:

(a) die Reaktion eines Säurechlorids der Formel

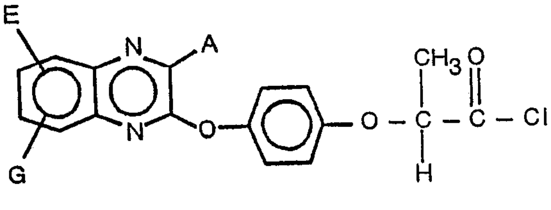

(IIc)

worin A, E und G wie vorstehend definiert sind, mit 2-(Ethoxyethoxy)ethanol in Anwesenheit einer äquimolaren Menge eines Säureakzeptors; oder

(b) die Reaktion einer Säure der Formel

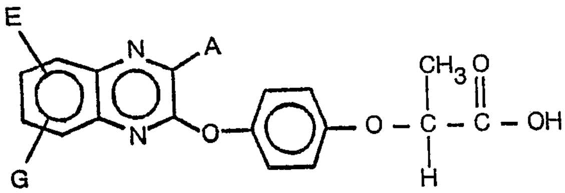

(IIb)

worin A, E und G wie vorstehend definiert sind, mit einem 1-Halogen-2-(ethoxyethoxy)ethan in Anwesenheit eines Phasentransfer-Katalysators.

2. Ein Verfahren nach Anspruch 1, worin A H ist.

3. Ein Verfahren nach Anspruch 2, worin G H ist und E H, Cl, F oder $CF_3$ ist, oder G und E 6- und 7-Cl sind.

4. Ein Verfahren nach Anspruch 3, worin G H und E H, Cl, F oder $CF_3$ ist.

5. Ein Verfahren nach Anspruch 4, worin E in der 6-Stellung ist.

6. Ein Verfahren nach Anspruch 1, worin das Produkt 2-(2-Ethoxyethoxy)ethyl-2-[4-(6-chlorchuinoxalin-2-yloxy)phenoxy]-propanoat ist.

7. Eine Zusammensetzung zur Bekämpfung unerwünschter Vegetation, umfassend eine herbizid wirksame Menge einer Verbindung der Formel (I), wie in einem der Ansprüche 1 bis 6 definiert, und mindestens eines von (a) einem oberflächenaktiven Mittel und (b) einem festen oder flüssigen Verdünnungsmittel.

8. Ein Verfahren zur Bekämpfung unerwünschter Vegetation, welches den Auftrag auf den Ort dieser Vegetation von einer herbizid wirksamen Menge einer Verbindung der Formel (I) wie in einem der Ansprüche 1 bis 6 definiert, umfaßt.

9. Das Verfahren nach Anspruch 8, worin die unerwünschte Vegetation in Reis selektiv bekämpft wird.

10. Das Verfahren nach Anspruch 8 oder 9, worin die Verbindung nach Anspruch 6 verwendet wird.

**Revendications pour les Etats contractants: CH GB IT LI LU SE**

1. Un composé de la formule

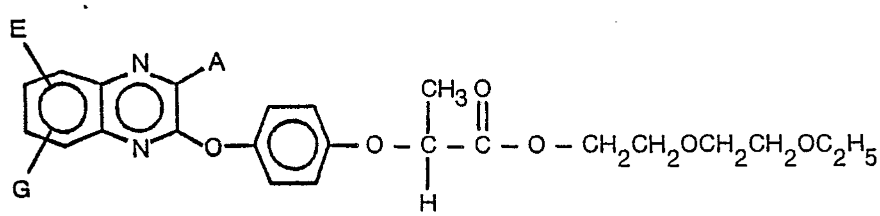

où A est H ou un radical alkyle en $C_1$—$C_4$;
E est H, Cl, Br, F, $CF_3$, $CH_3$ ou $OCH_3$;
G est H ou Cl;
avec les conditions que:
a) lorsque E n'est pas H ou Cl; alors G est H;
b) lorsque E ou G n'est pas H, alors A est H ou $CH_3$.

2. Un composé selon la revendication 1, dans lequel A est H.

3. Un composé selon la revendication 2, dans lequel G est H et E est H, Cl, F ou $CF_3$, ou G et E sont des atomes de chlore dans les positions 6 et 7.

4. Un composé selon la revendication 3, dans lequel G est H et E est H, Cl, F ou $CF_3$.

5. Un composé selon la revendication 4, dans lequel E est dans la position 6.

6. Un composé selon la revendication 1, qui est le 2-[4-(6-chlor-quinoxalin-2-yloxy)phénoxy]propanoate de 2-(2-éthoxyéthoxy)-éthyle.

7. Une composition pour lutter contre la végétation indésirable, comprenant une quantité efficace comme herbicide, d'un composé selon l'une quelconques des revendications 1 à 6 et au moins l'un des ingrédients suivants:
a) un agent tensio-actif et b) un diluant solide ou liquide.

8. Un procédé pour lutter contre la végétation indésirable, qui comprend l'application, à l'endroit de ladite végétation, d'une quantité efficace comme herbicide, d'un composé selon l'une quelconques des revendications 1 à 6.

9. Le procédé de la revendication 8, dans lequel on lutte contre de la végétation indésirable dans du riz.

10. Un procédé de préparation d'un composé selon l'une quelconques des revendications 1 à 6 qui consiste:

a) à faire réagir un chlorure d'acide de formule:

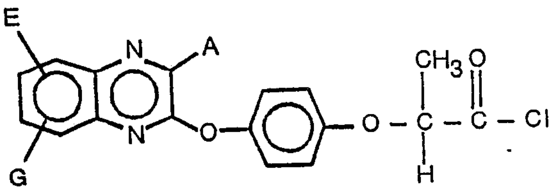

(IIc)

où A, E et G sont tels que définis dans la revendication 1, avec du 2-(éthoxyéthoxy)éthanol en présence d'une quantité équimolaire d'un accepteur d'acide; ou

b) à faire réagir un acide de formule

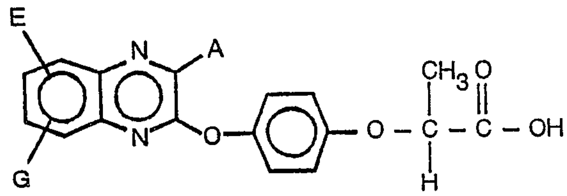

(IIb)

où A, E et G sont tels que définis dans la revendication 1, avec un 1-halogène-2-(éthoxyéthoxy)éthane en présence d'un catalyseur de transfert de phase.

**Revendications pour l'Etat contractant: AT**

1. Un procédé de préparation d'un composé de la formule

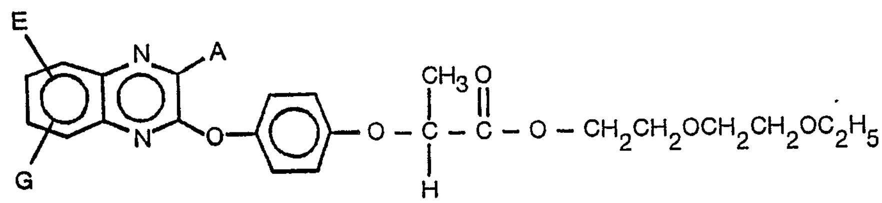

où A est H ou un radical alkyle en $C_1$—$C_4$;
E est H, Cl, Br, F, $CF_3$, $CH_3$ ou $OCH_3$;
G est H ou Cl;
avec les conditions que:
(a) lorsque E n'est pas H ou Cl; alors G est H;
(b) lorsque E ou G n'est pas H, alors A est H ou $CH_3$;
qui consiste:
(a) à faire réagir un chlorure d'acide de formule

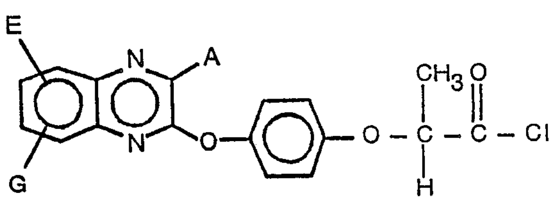

(IIc)

où A, E et G sont tels que définis dans la revendication 1, avec du 2-(éthoxyéthoxy)éthanol en présence d'une quantité équimolaire d'un accepteur d'acide; ou

(b) à faire réagir un acide de formule

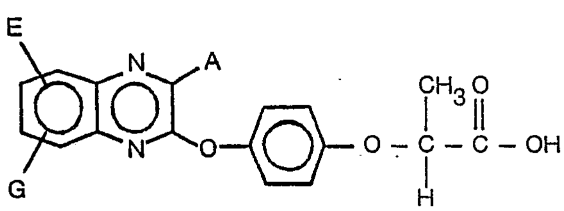

(IIb)

où A, E et G sont tels que définis dans la revendication 1, avec un 1-halogéno-2-(éthoxyéthoxy)éthane en présence d'un catalyseur de transfert de phase.

2. Un procédé selon la revendication 1, dans lequel A est H.

3. Un procédé selon la revendication 2, dans lequel G est H et E est H, Cl, F ou $CF_3$, ou G et E sont des atomes de chlore dans les positions 6 et 7.

4. Un procédé selon la revendication 3, dans lequel G est H et E est H, Cl, F ou $CF_3$.

5. Un procédé selon la revendication 4, dans lequel E est dans la position 6.

6. Un procédé selon la revendication 1, dans lequel le produit est le 2-[4-(6-chloroquinoxalin-2-yloxy)-phénoxy]propanoate de 2-(2-éthoxyéthoxy)-éthyle.

7. Une composition pour lutter contre la végatation indésirable, comprenant une quantité efficace comme herbicide, d'un composé de formule (I) tel que défini à l'une quelconque des revendications 1 à 6 et au moins l'un des ingrédients suivants: (a) un agent tensio-actif et (b) un diluant solide ou liquide.

8. Un procédé pour lutter contre la végétation indésirable qui comprend l'application, à l'endroit de ladite végétation, d'une quantité efficace comme herbicide, d'un composé de formule (I) tel que défini à l'une quelconque des revendications 1 à 6.

9. Le procédé de la revendication 8, dans lequel on lutte contre de la végétation indésirable dans du riz.

10. Le procédé de la revendication 8 ou 9, dans lequel on emploie le composé de la revendication 6.